# NEUE EUROPÄISCHE PATENTSCHRIFT

(11) **EP 2 556 050 B2**
(45) Veröffentlichungstag und Bekanntmachung des Hinweises auf die Entscheidung über den Einspruch: **06.09.2017**
(45) Hinweis auf die Patenterteilung: 14.05.2014
(21) Anmeldenummer: 11714959.1
(22) Anmeldetag: 06.04.2011
(51) Int. Cl.: C07C 253/30

(54) **VERFAHREN ZUR ISOMERISIERUNG VON CIS-2-PENTENNITRIL ZU 3-PENTENNITRILEN**
METHOD FOR ISOMERIZING CIS-2-PENTENE NITRILE TO FORM 3-PENTENE NITRILES
PROCÉDÉ D'ISOMÉRISATION DE CIS-2-PENTÈNENITRILE EN 3-PENTÈNENITRILE

(30) Priorität: 07.04.2010 EP 10159230
(43) Veröffentlichungstag der Anmeldung: 13.02.2013
(73) Patentinhaber: BASF SE, 67056 Ludwigshafen am Rhein (DE)
(72) Erfinder: LUYKEN, Hermann, 67069 Ludwigshafen (DE); PFAB, Peter, Shaker Heights OH 44122 (US); BAUMANN, Robert, 68529 Mannheim (DE); LEITNER, Andreas, A-4040 Linz (AT); AECHTNER, Tobias, 68165 Mannheim (DE)
(74) Vertreter: Féaux de Lacroix, Stefan
(86) Internationale Anmeldenummer: PCT/EP2011/055353
(87) Internationale Veröffentlichungsnummer: WO 2011/124610

(56) Entgegenhaltungen:
- WO-A1-2004/094364
- WO-A1-2005/073176
- US-A1- 2006 194 979
- US-A1- 2007 287 851
- PINES H. ET AL: 'Base-Catalyzed Reactions of Hydrocarbons and Related Compounds', 1977, ACADEMIC PRESS, NEW YORK Seiten 89 - 105
- PROCHÁZKA M. ET AL: 'Isomerisation of unsaturated nitriles' COLLECTION CZECHOSLOV. CHEM. COMMUN. Bd. 35, 1970, Seiten 1224 - 1234
- ATKINS P.W.: 'The Elements of Physical Chemistry', 1996, OXFORD UNIVERSITY PRESS Seite 107
- CLAYDEN J. ET AL: 'Organic Chemistry', 2001, OXFORD UNIVERSITY PRESS Seiten 197-199 - 227-232 UND 581-583
- LYNN J.W. ET AL: 'Syntesis and some derivatives of 3-Butenenitrile' J. ORG. CHEM. Bd. 26, 1961, Seiten 4300 - 4303
- SCRIVEN E. ET AL: 'Pyridine and Pyridine Derivatives', 02 Dezember 2005, KIRK-OTHMER ENCYCLOPEDIA OF CHEMICAL TECHNOLOGY Seiten 91 - 133
- HUBERT A.J. ET AL: 'The isomerization of Olefine part I. Base-catalysed isomerization of Olefins' SYNTHESIS 1969, Seiten 97 - 112
- BEYER-WALTER ET AL: 'Lehrbuch der Organischen Chemie', 2004, S. HIRZEL VERLAG, STUTTGART Seite 166

## Beschreibung

Die vorliegende Erfindung betrifft ein verbessertes Verfahren zur Isomerisierung von cis-2-Pentennitril zu 3-Pentennitrilen in Gegenwart von tertiären Aminen oder von Amidinen als Katalysatoren.

Aus der WO-A-05/73176 ist bekannt cis-2-Pentennitril mit Hilfe von homogen gelösten Aminen als Katalysatoren, ausgewählt aus der Gruppe C₁- bis C₂₀-Mono- und Diamine, zu 3-Pentennitrilen zu isomerisieren.

Aus der US-A-5 070 202 ist bekannt, dass cis-2-Pentennitril bei Temperaturen von 20 bis 200°C mit primären und sekundären Aminen Michael-Addukte bildet.

WO 2004/094364A1 beschreibt ein Verfahren zur isomerisierung von cis-2-Pentennitril zu 3-Pentennitrilen, wobei heterogene Katalysatoren verwendet werden.

Nachteilig an diesen Isomerisierungen ist, dass sich aus cis-2-Pentennitril und den genannten Aminen als Katalysatoren Michael-Addukte bilden.

Der vorliegenden Erfindung lag daher die Aufgabe zugrunde, den zuvor genannten Nachteilen abzuhelfen.

Demgemäß wurde ein neues und verbessertes Verfahren zur Isomerisierung von cis-2-Pentennitril zu 3-Pentennitrilen gefunden, welches dadurch gekennzeichnet ist, dass man cis-2-Pentennitril mit Amidinen, tertiären Aminen oder deren Gemischen als Katalysator bei Temperaturen von 80 bis 200°C und einem Druck von 0,01 bis 50 bar isomerisiert.

Das erfindungsgemäße Verfahren kann wie folgt durchgeführt werden:
Cis-2-Pentennitril kann mit einem Katalysator bei Temperaturen von 80 bis 200°C, bevorzugt 90 bis 150°C, besonders bevorzugt 100 bis 150°C und einem Druck von 0,01 bis 50 bar, bevorzugt 0,1 bis 30 bar, besonders bevorzugt 0,5 bis 20 bar, insbesondere Normaldruck (Atmosphärendruck) diskontinuierlich oder bevorzugt kontinuierlich umgesetzt werden.

Die tertiären Amine, bevorzugt die tertiären Alkyl-; Cycloalkyl-, Alkylcycloalkylamine und deren Gemische sollten gleich oder niedriger, also 0 bis 50°C, bevorzugt 1 bis 40°C, besonders bevorzugt 3 bis 30°C, insbesondere 4 bis 20°C, ganz besonders bevorzugt 5 bis 10°C niedriger als cis-2-Pentennitril (Siedepunkt 127°C/1013 mbar) oder höher, also 1 bis 50°C, bevorzugt 2 bis 30°C, besonders bevorzugt 3 bis 20°C, insbesondere 5 bis 10°C höher als cis-3-Pentennitril (Siedepunkt 146°C/1013 mbar) sieden.

Die Gemische aus tertiären Aminen und Amidinen sollten gleich oder niedriger, also 0 bis 50°C, bevorzugt 1 bis 40°C, besonders bevorzugt 3 bis 30°C, insbesondere 4 bis 20°C, ganz besonders bevorzugt 5 bis 10°C niedriger als cis-2-Pentennitril oder höher, also 1 bis 50°C, bevorzugt 2 bis 30°C, besonders bevorzugt 3 bis 20°C, insbesondere 5 bis 10°C höher als cis-3-Pentennitril sieden.

Die Aminopyridine, die Imidazole, die bicyclische Amidine II oder deren Gemische sollten höher, also 1 bis 50°C, bevorzugt 2 bis 30°C, besonders bevorzugt 3 bis 20°C, insbesondere 5 bis 10°C höher als cis-3-Pentennitril sieden.

Die Isomerisierung kann in beliebigen, geeigneten Apparaturen wie Rohrreaktoren oder Rührautoklaven, beispielsweise in einem Rührautoklaven drucklos, also bei Normaldruck (Atmosphärendruck) oder unter dem sich bei der jeweiligen Reaktionstemperatur einstellenden Druck durchgeführt werden.

Das Molverhältnis von Katalysator zu cis-2-Pentennitril kann in weiten Grenzen variiert werden und beträgt in der Regel 0,1:1 bis 1:1 Mol, bevorzugt 0,1:1 bis 0,5:1, besonders bevorzugt 0,15:1 bis 0,3:1 Mol.

Der Reaktionsaustrag der Isomerisierung kann destillativ aufgearbeitet werden. Nicht umgesetztes cis-2-Pentennitril kann als Kopfprodukt, gegebenenfalls zusammen mit niedriger als cis-2-Pentennitril siedendem Katalysator, in einem weiteren Isomerisierungsansatz wieder verwendet werden. Trans-3- und trans-2-Pentennitril können, gegebenenfalls nach Abtrennung und Rückführung von höher als cis-3-Pentennitril siedender Katalysatoren zur Pentennitril-Hydrocyanierung verwendet werden.

Als cis-2-Pentennitril eignen sich reines cis-2-Pentennitril, Mischungen, die cis-2-Pentennitril enthalten, oder Nebenproduktströme der Hydrocyanierung von 1,3-Butadien, die cis-2-Pentennitril, bevorzugt mehr als 50 Gew.-% cis-2-Pentennitril, besonders bevorzugt mehr als 50 Gew.-% cis-2-Pentennitril enthalten.

Als Katalysatoren für die Isomerisierung eignen sich Stickstoffbasen wie tertiäre Amine oder deren Gemische oder Amidine oder deren Gemische oder Gemische aus tertiären Aminen und Amidinen. Die Katalysatoren werden im Folgenden auch als Stickstoffbasen bezeichnet.

Als tertiäre Amine eignen sich tertiäre Alkyl-, Cycloalkyl- oder Alkylcycloalkylamine, Aminopyridine, Imidazole, oder deren Gemische, bevorzugt tertiäre Alkyl- oder Cycloalkylamine, Imidazole, oder deren Gemische, besonders bevorzugt tertiäre Alkylamine.

Als Amidine eignen sich bicyclische Amidine II.

Als tertiäre Amine eignen sich Amine der allgemeinen Formel I[R¹R²R³N], in der die Reste R¹, R², R³ unabhängig voneinander C₁- bis C₂₀-Alkyl, bevorzugt C₁- bis C₈-Alkyl, besonders bevorzugt C₁- bis C₆-Alkyl wie Methyl, Ethyl, n-Propyl, iso-Propyl, n-Butyl, iso-Butyl, sec-Butyl, tert.-Butyl, n-Pentyl, iso-Pentyl, n-Hexyl, iso-Hexyl, C₃- bis C₁₂-Cycloalkyl, bevorzugt C₄- bis C₈-Cycloalkyl, besonders bevorzugt C₅- bis C₇-Cycloalkyl wie Cyclopentyl, Cyclohexyl und Cycloheptyl, mit C₁- bis C₄-Alkyl wie Methyl, Ethyl, n-Propyl, iso-Propyl, n-Butyl, iso-Butyl, sec-Butyl, tert.-Butyl, ein- bis dreifach substituiertes C₃- bis C₁₂-Cycloalkyl, bevorzugt C₄- bis C₈-Cycloalkyl, besonders bevorzugt C₅- bis C₇-Cy_{d}oalkyl wie Cyclopentyl, Cyclohexyl, Cycloheptyl oder deren Gemische oder zwei der Reste R¹, R², R³ zusammen eine gegebenenfalls durch C₁- bis C₄-Alkyl wie Methyl, Ethyl, n-Propyl, iso-Propyl, n-Butyl, iso-Butyl, sec-Butyl, tert-Butyl, substituierte lineare C₄- bis C₉-Kette, bevorzugt C₅- bis C₇-Kette, die zusätzlich ein Stickstoff-, Sauerstoff- oder Schwefelatom enthalten können, bedeuten. Die Reste R¹, R², R³ können aber auch so verknüpft sein, dass ein tertiäres Amin entsteht, das unabhängig voneinander zwei fünf- bis siebengliedrige Ringe enthält.

Als tertiäre Amine der Formel I sind beispielsweise Trimethylamin, Triethylamin, Methyldiethylamin, Dimethylethylamin, Tributylamin, Ethyldipropylamin, Tripropylamin, Trihexylamin, Tricyclohexylamin, Ethyldicyclöhexylamin, N-Methylpiperidin, N-Ethylpiperidin, N-Methylpyrrolidin, N-Ethylpyrrolidin, N-Propylazepin, N-Ethylmorpholin, N.N'-Dimethylpiperazin oder Gemische dieser tertiären Amine, bevorzugt Triethylamin geeignet.

Als tertiäre Amine der Formel I, die unabhängig voneinander zwei fünf- bis siebengliedrige Ringe enthalten, eignen sich beispielsweise Tropan, Granatan und Chinuclidin.

Als tertiäre Amine sind weiterhin 4-Aminopyridine, wobei die beiden Wasserstoffatome der Aminogruppe die gleichen Bedeutungen haben können, wie die Reste R¹, R², R³.

Als 4-Aminopyridine eignen sich beispielsweise N,N-Dimethyl-4-aminopyridin, N,N-Diethyl-4-aminopyridin, 4-Morpholinopyridin oder 4-Piperazinopyridin, bevorzugt N,N-Dimethyl-4-aminopyridin oder N,N-Diethyl-4-aminopyridin, besonders bevorzugt N,N-Dimethyl-4-aminopyridin.

Als tertiäre Amine eignen sich weiterhin N-substituierte Imidazole, wobei der Substituent C₁- bis C₂₀-Alkyl, bevorzugt C₁-bis C₈-Alkyl wie Methyl, Ethyl, n-Propyl, iso-Propyl, n-Butyl, iso-Butyl, sec-Butyl, tert.-Butyl, n-Pentyl, iso-Pentyl, n-Hexyl, iso-Hexyl, besonders bevorzugt C₁- bis C₄-Alkyl wie Methyl, Ethyl, n-Propyl, iso-Propyl, n-Butyl, iso-Butyl, sec-Butyl oder tert.-Butyl, insbesondere C₁- bis C₂-Alkyl wie Methyl oder Ethyl bedeutet.

Geeignete Imidazole sind beispielsweise N-Methyl-, N-Ethyl- oder N-Propylimidazol.

Als Amidine eignen sich beispielsweise bicyclische Amidine der Formel II 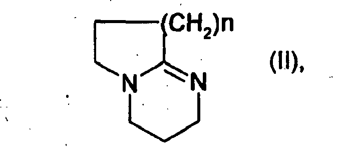 in der n 1, 2 oder 3, bevorzugt 1 oder 3, besonders bevorzugt 1 bedeutet. Beispiele hierfür sind 1.5-Diazabicyclo[4.3.0]-5-nonen (DBN) und 1.8-Diazabicyclo[5.4.0]-7-undecen (DBU).

Cis-2-Pentennitril, das Ausgangsprodukt für die Isomerisierung zu 3-Pentennitrilen, entsteht beispielsweise bei der Hydrocyanierung von 3-Pentennitrilen zu Adipodinitril.

Die zweistufige Herstellung von Adipodinitril, ausgehend von Butadien und Blausäure ist bekannt (Hans-Jürgen Arpe, Industrielle Organische Chemie, 6. Auflage 2007, Wiley-VCH Verlag, Seiten 272 bis 273):
Im ersten Reaktionsschritt wird Butadien in der Flüssigphase in Gegenwart von Nickel(0)-tritolylphosphit-Komplexverbindungen als Katalysatoren hydrocyaniert. Man isoliert ein Gemisch isomerer Pentennitrile und Methylbutennitrile, insbesondere 3-Pentennitrile und 2-Methyl-3-butennitrile. Die 2-Methyl-3-butennitrile werden zu 3-Pentennitrilen isomerisiert.

Im zweiten Reaktionsschritt wird 3-Pentennitril in der Flüssigphase mit Blausäure hydrocyaniert. Man arbeitet in Gegenwart der gleichen Nickel(0)-tritolylphosphit-Komplexe, denen eine Lewis-Säure wie z. B. Zinkchlorid hinzugefügt wird.

Aus dem Hydrocyanierungs-Austrag werden Adipodinitril, weitere Dinitrile und der Nickel(0)-Katalysator-Komplex abgetrennt. Der Katalysator wird, gegebenenfalls nach Regenerierung, in die Hydrocyanierung zurückgeführt.

Die bei der Aufarbeitung erhaltene organische Phase enthält im Wesentlichen ungesättigte C₅-Mononitrile, ausgewählt aus der Gruppe cis-2-Pentennitril, trans-3-Pentennitril, cis-3-Pentennitril, 4-Pentennitril, trans-2-Pentennitril, cis-2-Methyl-2-butennitril, trans-2-Methyl-2-butennitril, 2-Methyl-3-butennitril.

Dieser C₅-Mononitrilstrom kann 0,1 bis 10 Gew.-% cis-2-Pentennitril, insbesondere 1 bis 5 Gew.-% cis-2-Pentennitril enthalten.

Für die diskontinuierliche Isomerisierung kann ein derart gewonnenes cis-2-Pentennitril zunächst destillativ aus dem C₅-Mononitril-Gemisch abgetrennt werden. Für die Isomerisierung kann ein cis-2-Pentennitril mit einer Reinheit von > 50 Gew.-%, bevorzugt > 70 Gew.-%, eingesetzt werden.

In einer besonders bevorzugten Ausführungsform kann die Isomerisierung kontinuierlich in integrierter Fahrweise mit im Vergleich zu cis-2-Pentennitril gleich oder niedriger siedenden Stickstoffbasen als Katalysatoren durchgeführt werden. Unter integrierter Fahrweise ist dabei zu verstehen, dass cis-2-Pentennitril, Gemische aus 3-Pentennitril und trans-2-Pentennitril und die Stickstoffbase kontinuierlich in die jeweiligen Verfahrensstufen zurückgeführt werden, das heißt, dass alle Rückführungs-Ströme geschlossen sind.

Diese Aufgabe wird gelöst durch ein kontinuierliches, integriertes Verfahren zur Isomerisierung von cis-2-Pentennitril zu 3-Pentennitrilen, wobei man
a) 3-Pentennitrile oder eine Mischung enthaltend 3-Pentennitrile in Gegenwart von Nickei(0)-Phosphorligand-Komplexen'als Katalysatoren zu Adipodinitril hydrocyaniert,
b) aus dem Hydrocyanierungsaustrag Adipodinitril, 2-Methylglutarnitril und Nickel(0)-Phosphorligand-Komplex abtrennt,
c) das cis-2-Pentennitril in der so erhaltenen, im wesentlichen ungesättigte C5-Mononitrile wie cis- und trans-3-Pentennitril, 4-Pentennitril, trans-2-Pentennitril, cis-2-Methyl-2-butennitril, trans-2-Methyl-2-butennitril, 2-Methyl-3-butennitril, enthaltenen organischen Phase mit Hilfe einer Stickstoffbase der Formel [R¹ R² R³N], in der die Reste R¹ R² R³ unabhängig voreinander C₁- bisC₂₀-Alkyl sind als Katalysator, die bei Normaldruck gleich oder niedriger als cis-2-Pentennitril siedet, zu 3-Pentennitrilen isomerisiert,
d) die in c) erhaltene, im wesentlichen ungesättigte C5-Mononitrile enthaltende organische Phase einer Destillationskolonne K1 zuführt, aus der Destillationskolonne K1 über Kopf cis-2-Pentennitril,cis- und trans-2-Methyl-2-butennitril, über Sumpf 3-Pentennitril und trans-2-Pentennitril abtrennt und in den Reaktionsschritt der Hydrocyanisierung von 3-Pentennitrilen zurückführt,
e) das Kopfprodukt der Kolonne K1 einer Destillationskolonne K2 zuführt, als Sumpfprodukt der Kolonne K2 trans-2-Methyl-2-butennitril abtrennt und ausschleust,
f) das Kopfprodukt der Kolonne K2 und eine gleich oder niedriger als cis-2-Pentennitril siedende Stickstoffbase einem Reaktor R1 zuführt, das Reaktionsprodukt des Reaktors R1 und das gleich oder niedriger als cis-2-Pentennitril siedende tertiäre Amin einer Destillationskolonne K3 zuführt
g) das Kopfprodukt der Destillationskolonne K3, das überwiegend aus dem tertiären Amin besteht, in den Reaktor R1 zurückführt und einen Teilstrom des Kopfprodukts, der cis-2-Methly-2-butennitril, tertiäres Amin und cis 2-Pentennitril enthält, ausschleust, aus einem Seitenabzug ein Gemisch aus cis-2-Pentennitril, trans-3-Pentennitril, trans-2-Pentennitrit und tertiärer Base in die Destillationskolonne K1 zurückführt und als Sumpfprodukt Hochsieder ausschleust.

Die kontinuierliche Isomerisierung von cis-2-Pentennitril zu 3-Pentennitrilen unter Verwendung von niedriger als 2-cis-Pentennitril siedenden tertiären Aminen I können in, dem Fachmann bekannten, Vorrichtung durchgeführt werden. Für die Destillation geeignet sind Vorrichtungen, wie sie beispielsweise in: Kirk-Othmer, Encyclopedia of Chemical Technology, 4. Ed., Vol. 8, John Wiley & Sons, New York, 1996, Seiten 334 bis 338, beschrieben sind, wie Siebbodenkolonnen, Glockenbodenkolonnen, Packungskolonnen, Füllkörperkolonnen, die auch als Trennwandkolonnen betrieben werden können. Diese Destillationsvorrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer sowie mit Vorrichtungen zur Kondensation des Brüdenstroms ausgerüstet. Die Destillation kann dabei in mehreren, wie zwei oder drei Vorrichtungen, durchgeführt werden. Die Destillation kann zudem einstufig im Sinne einer Teilverdampfung des Zulaufstroms erfolgen.

Die Schritte a) bis c) wurden schon auf den Seiten 3 und 4 beschrieben.

### Schritt d):

In Schritt d) wird die in Schritt c) erhaltene, im wesentlichen C₅-Mononitrile enthaltende organische Phase einer Destillationskolonne K1 zugeführt, die 20 bis 40 theoretische Böden besitzt und bei einer Sumpftemperatur im Bereich von 70 bis 145°C und einem Druck von 100 bis 1000 mbar betrieben wird.

Über Kopf der Kolonne K1 gehen cis-2-Pentennitril, cis- und trans-2-Methyl-2-butennitril und rückgeführtes tertiäres Amin I. Als Sumpfprodukte werden 3-Pentennitrile und trans-2-Pentennitril abgezogen und in Schritt a) zurückgeführt.

Es kann vorteilhaft sein, aus einem Seitenabzug 3-Pentennitrile und trans-2-Pentennitril abzuziehen, in Schritt a) zurückzuführen und gegebenenfalls Hochsieder als Sumpfprodukte auszuschleusen.

### Schritt e):

Das Kopfprodukt der Kolonne K1 wird einer Kolonne K2 zugeführt, die 20 bis 40 theoretische Böden besitzt und bei einer Sumpftemperatur im Bereich von 60 bis 140°C und einem Druck von 50 bis 100 mbar betrieben wird.

Das Kopfprodukt der Kolonne K2 enthält cis-2-Pentennitril,cis-2-Methyl-2-butennitril und rückgeführtes tertiäres Amin I. Als Sumpfprodukt wird trans-2-Methyl-2-butennitril ausgeschleust.

### Schritt f):

Das Kopfprodukt der Kolonne K2 wird einem Reaktor R1 zugeführt, in dem der überwiegende Teil der Isomerisierung erfolgt. Außerdem wird der Kolonne K2 soviel tertiäres Amin I zugeführt, dass Amin-Verluste im Kreislauf, die durch Ausschleusung zustande kommen, ausgeglichen werden.

Die erfindungsgemäße Isomerisierung kann in jeder geeigneten, dem Fachmann bekannten Apparatur durchgeführt werden. Für die Reaktion kommen somit übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055, beschrieben sind, wie Rührkesselreaktoren, Schlaufenreaktoren, Gasumlaufreaktoren, Blasensäulenreaktoren oder Rohrreaktoren, bevorzugt Rohrreaktoren, jeweils gegebenenfalls mit Vorrichtungen zur Wärmeübertragung. Die Reaktion kann in mehreren, wie zwei oder drei, Apparaturen durchgeführt werden.

Besonders bevorzugt ist eine Rührkesselkaskade mit 2 bis 4, insbesondere 3 Rührkessein oder ein Strömungsrohr.

Der Isomerisierungsreaktor wird bei Temperaturen von 100 bis 150°C, bevorzugt 100 bis 130°C und Drucken von 1 bis 20 bar betrieben. Die Verweilzeiten im Reaktor betragen 0.5 bis 5 Stunden, bevorzugt 1 bis 3 Stunden. Der cis-2-Pentennitril-Umsatz beträgt dabei 5 bis 30, bevorzugt 10 bis 25, besonders bevorzugt 10 bis 20 %.

### Schritt g):

Der Reaktionsaustrag des Reaktors R1, der 3-Pentennitrile als Isomerisierungsprodukte, trans-2-Pentennitril, cis-2-Methyl-2-butennitril, nicht umgesetztes cis-2-Pentennitril und tertiäres Amin I enthält, wird einer Kolonne K3 zugeführt. Die Kolonne K3 besitzt 10 bis 20 theoretische Böden und wird bei einer Sumpftemperatur im Bereich von 100 bis 150°C und einem Druck von 100 bis 1000 mbar, betrieben.

Das Kopfprodukt der Kolonne K3, das überwiegend aus dem tertiären Amin I besteht, wird in den Reaktor R1 zurückgeführt. Ein Teilstrom des Kopfproduktes wird ausgeschleust, um eine Aufpegelung von cis-2-Methyl-2-butennitril zu verhindern. Aus einem Seitenabzug wird ein Strom entnommen, der neben überwiegend cis-2-Pentennitril und tertiärem Amin I trans-3-Pentennitril, trans-2-Methyl-2-butennitril enthält. Dieser Strom wird auf die Kolonne K1 zurückgeführt. Als Sumpfprodukt werden Hochsieder ausgeschleust.

Es ist weiterhin möglich, in Schritt f) Stickstoffbasen als Katalysatoren für die Isomerisierung von cis-2-Pentennitril zu 3-Pentennitrilen zu verwenden, die bei Normaldruck höher, insbesondere mindestens 5 bis 10°C höher als cis-3-Pentennitril sieden. Sie können beispielsweise zusammen mit Hochsiedern als Sumpfprodukt der Kolonne K3 entnommen und, gegebenenfalls nach Abtrennung der Hochsieder, z. B. in einer Kolonne oder einem Dünnschichtverdampfer, in den Reaktor R1 zurückgeführt werden.

In einer bevorzugten Ausführungsform der Erfindung, die für bei Normaldruck niedriger als cis-2-Pentennitril oder bei Normaldruck höher, insbesondere mindestens 5 bis 10°C höher als cis-3-Pentennitril siedende Stickstoffbasen anwendbar ist, wird der Reaktor R1 durch eine Reaktionskolonne ersetzt. In WO-A-05/73177 sind Reaktionskolonnen für die Isomerisierung von cis-2-Pentennitril zu 3-Pentennitrilen in Gegenwart von homogenen und heterogenen Katalysatoren beschrieben. Diese Anmeldung wird vollständig in die vorliegende Erfindung einbezogen.

Das erfindungsgemäße Verfahren wird vorzugsweise in einer Destillationskolonne, mindestens umfassend eine Sumpfzone, eine Reaktionszone und eine Kopfzone, durchgeführt. Dabei sind Sumpfzone, Reaktionszone und Kopfzone in der vorgegebenen Reihenfolge von unten nach oben in der Destillationskolonne angeordnet. Es ist dabei nicht ausgeschlossen, dass auch in der Sumpf- oder Kopfzone noch Reaktion stattfindet.

Zusätzlich kann die Destillationskolonne Einbauten mit destillativer Trennwirkung umfassen. Diese zusätzlichen Einbauten sind vorzugsweise unter- und/oder oberhalb der Reaktionszone angeordnet. In der unteren Trennzone, d.h. der Trennzone unterhalb der Reaktionszone, wird das schwer siedende Isomerisierungsprodukt weitgehend von leicht siedenden Komponenten abgetrennt. So werden z. B. trans-2-Pentennitril und trans-3-Pentennitril von nicht umgesetztem cis-2-Pentennitril getrennt. In der oberen Trennzone, d.h. der Trennzone oberhalb der Reaktionszone, werden leicht siedende Nebenkomponenten weitgehend von schwer siedenden Komponenten abgetrennt. So wird hier beispielsweise gegebenenfalls mit dem Eduktstrom eingetragenes trans-2-Methyl-2-butennitril von trans-3-Pentennitril und trans-2-Pentennitril getrennt. Ebenso kann aus nicht isomerisiertem cis-2-Pentennitril trans-3-Pentennitril und trans-2-Pentennitril abgereichert werden. Diese Trennungen sind nur beispielhaft aufgeführt und sind nicht einschränkend.

Bei optimaler Kolonnenkonfiguration kann so das gesamte cis-2-Pentennitril des Eduktstroms ohne zusätzlichen Reaktor umgesetzt werden und das gesamte trans-3-Pentennitril im Sumpf ohne zusätzlichen Trennapparat erhalten werden. Dabei sind die zusätzlichen Einbauten mit destillativer Trennwirkung (Trennzonen) im Allgemeinen von Vorteil, aber nicht zwingend notwendig. So kann auch eine der beiden oder beide Trennzonen entfallen.

Die Reaktionszone besteht im Allgemeinen aus mehreren unterschiedlichen Teilbereichen mit unterschiedlichen Funktionen. Die Teilbereiche unterscheiden sich durch die Aufgabe des Transportes von Gas zum Kopf der Kolonne und die Aufgabe des Ableitens von Flüssigkeit in Richtung des Kolonnensumpfes. Zudem können Flüssigkeitsverteiler innerhalb der Reaktionszone notwendig sein, um eine optimale Verteilung von Flüssigkeit über den Kolonnenquerschnitt zu gewährleisten. Auch Einbauten zum Eintragen von Wärme in die Kolonne können sich in der Reaktionszone befinden.

### Beispiele

### Beispiel 1

### Isomerisierung von cis-2-Pentennitril mit Triethylamin als Katalysator

Das als Einsatzstoff verwendete C₅-Mononitril-Gemisch wurde nach WO-A-05/73172, Beispiel 1, durch Hydrocyanierung von 3-Pentennitril in Gegenwart von Ni(0)Komplexen, die ausgehend von einer Ligandmischung aus 60 mol-% Tri(m/p-tolyl)phosphit und 40 mol-% des Chelatphosphoniten 1 synthetisiert worden waren, und Zinkchlorid hergestellt. Nach Abtrennung von Adipodinitril, weiterer Dinitrile und des Ni(0)-Katalysators wurde ein C₅-Nitril-Gemisch erhalten, das neben trans-3-Pentennitril (trans-3PN), cis-3-Pentennitril (cis-3PN) und 4-Pentennitril (4PN) weiterhin cis-2-Methyl-2-butennitril (cis-2M2BN) (5 %), trans-2-Methyl-2-butennitril (trans-2M2BN) (2 %), cis-2-Pentennitril (cis-2PN) (5 %) und trans-2-Pentennitril (trans-2PN) enthielt. Die Prozentangaben beziehen sich auf die Summe aller genannten ungesättigten Nitrile.

Das C₅-Gemisch wurde einer Kolonne K1 zugeführt, die 30 theoretische Trennstufen besaß und bei einer Sumpftemperatur von 79°C und einem Druck von 100 mbar betrieben wurde. Über Kopf gingen cis-2-Pentennitril, cis- und trans-2-Methyl-2-butennitril und aus Kolonne 3 rückgeführtes Triethylamin. Als Sumpfprodukte wurden 3-Pentennitril und trans-2-Pentennitril abgezogen und in den Schritt a), die Hydrocyanierung von 3-Pentennitril, zurückgeführt.

Das Kopfprodukt der Kolonne K1, das 79 Gew.-% cis-2-Pentennitril und 10 Gew.-% 2-Methyl-2-butennitrile enthielt, wurde der Kolonne 2 zugeführt, die 33 theoretische Trennstufen besaß und bei einer Sumpftemperatur von 72°C und einem Druck von 100 mbar betrieben wurde.

Das Kopfprodukt der Kolonne K2 enthielt 86 Gew.-% cis-2-Pentennitril und 3 Gew.-% trans-2-Methyl-2-butennitril. Als Sumpfprodukt der Kolonne K2 wurde ein Strom ausgeschleust, der 61 Gew.-% trans-2-Methyl-2-butennitril enthielt.

Das Kopfprodukt der Kolonne K2 wurde einem Reaktorsystem RS-1 zugeführt, das aus einer Kaskade von drei Rührkesseln bestand. In RS-1 fand der überwiegend Teil der cis-2-Pentennitril-Isomerisierung bei einer Verweilzeit von 2 Stunden statt. Triethylamin-Verluste, z.B. durch Ausschleusung, konnten über eine Triethylamin-Zuleitung ausgeglichen werden. Die Isomerisierungstemperatur betrug 125°C, der Druck 2 bar. Das Molverhältnis von cis-2-Pentennitril zu Triethylamin lag bei 1 zu 0,5, der cis-2-Pentennitril-Umsatz beträgt 13 %.

Der Reaktionsaustrag des Reaktorsystems RS-1 wurde einer Kolonne K3 zugeführt, die 15 theoretische Trennstufen besaß und bei einer Sumpftemperatur von 110°C und einem Druck von 250 mbar betrieben wurde.

Das Kopfprodukt der Kolonne K3, das zu 95 Gew.-% aus Triethylamin bestand, wurde in das Reaktorsystem zurückgeführt. Ein Teilstrom, der 6 Gew.-% des Kopfprodukts ausmachte, wurde ausgeschleust. Ein Seitenabzugsprodukt, das zu 79 Gew.-% aus cis-2-Pentennitril bestand, wurde auf die Kolonne K2 zurückgeführt. Über Sumpf wurden Hochsieder ausgeschleust.

## Patentansprüche

1. Verfahren zur Isomerisierung von cis-2-Pentennitril zu 3-Pentennitrilen, **dadurch gekennzeichnet, dass** man cis-2-Pentennitril mit tertiären Aminen oder deren Gemischen als Katalysator bei Temperaturen von 80 bis 200°C und einem Druck von 0,01 bis 50 bar isomerisiert.

2. Verfahren zur Isomerisierung von cis-2-Pentennitril zu 3-Pentennitrilen nach Anspruch 1, **dadurch gekennzeichnet, dass** man als Katalysatoren tertiäre Alkyl-, Cycloalkyl-, Alkylcycloalkylamine, Aminopyridine, Imidazole oder deren Gemische einsetzt.

3. Verfahren zur Isomerisierung von cis-2-Pentennitril zu 3-Pentennitrilen nach Anspruch 1 oder 2, **dadurch gekennzeichnet, dass** man als Katalysator N,N-Dimethyl-4-aminopyridin einsetzt.

4. Verfahren zur Isomerisierung von cis-2-Pentennitril zu 3-Pentennitrilen nach einem der Ansprüche 1 bis 3, **dadurch gekennzeichnet, dass** man als Katalysatoren Triethylamin einsetzt.

5. Verfahren zur Isomerisierung von cis-2-Pentennitril zu 3-Pentennitrilen nach einem der Ansprüche 1 bis 4, **dadurch gekennzeichnet, dass** man Katalysatoren einsetzt, deren Siedepunkt gleich oder niedriger, also 0 bis 50°C niedriger als cis-2-Pentennitril oder höher, also 1 bis 50°C höher als cis-3-Pentennitril liegt.

6. Verfahren zur Isomerisierung von cis-2-Pentennitril zu 3-Pentennitrilen nach einem der Ansprüche 1 bis 5, **dadurch gekennzeichnet, dass** man die Isomerisierung von cis-2-Pentennitril zu 3-Pentennitrilen in einer Reaktionskolonne durchführt.

7. Verfahren zur kontinuierlichen Isomerisierung von cis-2-Pentennitril zu 3-Pentennitrilen nach Anspruch 1, wobei man
a) 3-Pentennitrile oder eine Mischung enthaltend 3-Pentennitrile in Gegenwart von Nickel(0)-Phosphorligand-Komplexen als Katalysatoren zu Adipodinitril hydrocyaniert,
b) aus dem Hydrocyanierungsaustrag Adipodinitril, 2-Methylglutarnitril und Nickel(0)-Phosphorligand-Komplex abtrennt,
c) das cis-2-Pentennitril in der so erhaltenen, im wesentlichen ungesättigte C5-Mononitrile wie cis- und trans-3-Pentennitrile, 4-Pentennitril, trans-2-Pentennitril, cis-2-Methyl-2-butennitril, trans-2-Methyl-2-butennitril, 2-Methyl-3-butennitril, enthaltenden organischen Phase mit Hilfe einer Stickstoffbase der Formel [R¹R²R³N], in der die Reste R¹, R², R³ unabhängig voneinander C₁- bis C₂₀-Alkyl sind, als Katalysator, die bei Normaldruck gleich oder niedriger als cis-2-Pentennitril siedet, zu 3-Pentennitrilen isomerisiert, **dadurch gekennzeichnet, dass** man
d) die in c) erhaltene, im Wesentlichen ungesättigte C5-Mononitrile enthaltende organische Phase einer Destillationskolonne K1 zuführt, aus der Destillationskolonne K1 über Kopf cis-2-Pentennitril, cis- und trans-2-Methyl-2-butennitril, über Sumpf 3-Pentennitril und trans-2-Pentennitril abtrennt und in den Reaktionsschritt der Hydrocyanisierung von 3-Pentennitrilen zurückführt,
e) das Kopfprodukt der Kolonne K1 einer Destillationskolonne K2 zuführt, als Sumpfprodukt der Kolonne K2 trans-2-Methyl-2-butennitril abtrennt und ausschleust,
f) das Kopfprodukt der Kolonne K2 und ein bei Normaldruck gleich oder niedriger als cis-2-Pentennitril siedenden Stickstoffbase einem Reaktor R1 zuführt, das Reaktionsprodukt des Reaktors R1 und das gleich oder niedriger als cis-2-Pentennitril siedende tertiäre Amin einer Destillationskolonne K3 zuführt,
g) das Kopfprodukt der Destillationskolonne K3, das überwiegend aus dem tertiären Amin besteht, in den Reaktor R1 zurückführt und einen Teilstrom des Kopfprodukts, der cis-2-Methyl-2-butennitril, tertiäres Amin und cis-2-Pentennitrill enthält, ausschleust, aus einem Seitenabzug ein Gemisch aus cis-2-Pentennitril, trans-3-Pentennitril, trans-2-Pentennitril und tertiärer Base in Destillationskolonne K1 zurückführt und als Sumpfprodukt Hochsieder ausschleust.

8. Verfahren zur kontinuierlichen Isomerisierung von cis-2-Pentennitril zu 3-Pentennitrilen nach Anspruch 7, **dadurch gekennzeichnet, dass** man die Isomerisierung von cis-2-Pentennitril zu 3-Pentennitrilen in einer Reaktionskolonne durchführt.

9. Verwendung von tertiären Aminen oder deren Gemischen als Katalysatoren für die Isomerisierung von cis-2-Pentennitril zu 3-Pentennitril.

## Claims

1. A process for isomerizing cis-2-pentenenitrile to 3-pentenenitriles, which comprises isomerizing cis-2-pentenenitrile with tertiary amines or mixtures thereof as a catalyst at temperatures of 80 to 200°C and a pressure of 0.01 to 50 bar.

2. The process for isomerizing cis-2-pentenenitrile to 3-pentenenitriles according to claim 1, wherein the catalysts used are tertiary alkyl-, cycloalkyl-, alkylcycloalkylamines, aminopyridines, imidazoles or mixtures thereof.

3. The process for isomerizing cis-2-pentenenitrile to 3-pentenenitriles according to claim 1 or 2, wherein the catalyst used is N,N-dimethyl-4-aminopyridine.

4. The process for isomerizing cis-2-pentenenitrile to 3-pentenenitriles according to any of claims 1 to 3, wherein the catalyst used is triethylamine.

5. The process for isomerizing cis-2-pentenenitrile to 3-pentenenitriles according to any of claims 1 to 4, wherein the catalysts used have boiling points equal to or lower than, i. e. 0 to 50 °C lower than, that of cis-2-pentenenitrile, or higher than, i.e. 1 to 50°C higher than, that of cis-3-pentenenitrile.

6. The process for isomerizing cis-2-pentenenitrile to 3-pentenenitriles according to any of claims 1 to 5, wherein the isomerization of cis-2-pentenenitrile to 3-pentenenitriles is performed in a reaction column.

7. The process for continuously isomerizing cis-2-pentenenitrile to 3-pentenenitriles according to claim 1, by
a) hydrocyanating 3-pentenenitriles or a mixture comprising 3-pentenenitriles to adiponitrile in the presence of nickel(0)-phosphorus ligand complexes as catalysts,
b) removing adiponitrile, 2-methylglutaronitrile and nickel(0)-phosphorus ligand complex from the hydrocyanation output,
c) isomerizing the cis-2-pentenenitrile in the organic phase thus obtained, comprising essentially unsaturated C5-mononitriles such as cis- and trans-3-pentenenitriles, 4-pentenenitrile, trans-2-pentenenitrile, cis-2-methyl-2-butenenitrile, trans-2-methyl-2-butenenitrile, 2-methyl-3-butenenitrile, to 3-pentenenitriles with the aid of a nitrogen base of the formula [R¹R²R³N] in which the R¹, R², R³ radicals are each independently C₁- to C₂₀-alkyl, as a catalyst, the boiling point of which is equal to or lower than that of cis-2-pentenenitrile at standard pressure, wherein
d) the organic phase which comprises essentially unsaturated C5-mononitriles and is obtained in c) is fed to a distillation column K1, cis-2-pentenenitrile and cis- and trans-2-methyl-2-butenenitrile are removed via the top of the distillation column K1, and 3-pentenenitrile and trans-2-pentenenitrile via the bottom, and the latter is recycled into the reaction step for hydrocyanation of 3-pentenenitriles,
e) the top product of column K1 is fed to a distillation column K2, and trans-2-methyl-2-butenenitrile is removed and discharged as the bottom product of column K2,
f) the top product of column K2 and a nitrogen base having a boiling point equal to or lower than that of cis-2-pentenenitrile at standard pressure are fed to a reactor R1, and the reaction product of the reactor R1 and the tertiary amine having a boiling point equal to or lower than that of cis-2-pentenenitrile are fed to a distillation column K3,
g) the top product of the distillation column K3, which consists predominantly of the tertiary amine, is recycled into the reactor R1 and a substream of the top product comprising cis-2-methyl-2-butenenitrile, tertiary amine and cis-2-pentenenitrile is discharged, a mixture of cis-2-pentenenitrile, trans-3-pentenenitrile, trans-2-pentenenitrile and tertiary base is recycled from a side draw into distillation column K1, and high boilers are discharged as the bottom product.

8. The process for continuously isomerizing cis-2-pentenenitrile to 3-pentenenitriles according to claim 7, wherein the isomerization of cis-2-pentenenitrile to 3-pentenenitriles is performed in a reaction column.

9. The use of tertiary amines or mixtures thereof as catalysts for the isomerization of cis-2-pentenenitrile to 3-pentenenitrile.

## Revendications

1. Procédé d'isomérisation de cis-2-pentène-nitrile en 3-pentène-nitriles, **caractérisé en ce que** le cis-2-pentène-nitrile est isomérisé avec des amines tertiaires ou leurs mélanges en tant que catalyseur à des températures de 80 à 200 °C et à une pression de 0,01 à 50 bar.

2. Procédé d'isomérisation de cis-2-pentène-nitrile en 3-pentène-nitriles selon la revendication 1, **caractérisé en ce que** des alkyl-, cycloalkyl-, alkylcycloalkylamines, des aminopyridines, des imidazoles ou leurs mélanges sont utilisés en tant que catalyseurs.

3. Procédé d'isomérisation de cis-2-pentène-nitrile en 3-pentène-nitriles selon la revendication 1 ou 2, **caractérisé en ce que** la N,N-diméthyl-4-aminopyridine est utilisée en tant que catalyseur.

4. Procédé d'isomérisation de cis-2-pentène-nitrile en 3-pentène-nitriles selon l'une quelconque des revendications 1 à 3, **caractérisé en ce que** la triéthylamine est utilisée en tant que catalyseur.

5. Procédé d'isomérisation de cis-2-pentène-nitrile en 3-pentène-nitriles selon l'une quelconque des revendications 1 à 4, **caractérisé en ce que** des catalyseurs dont le point d'ébullition est inférieur ou égal, c'est-à-dire inférieur de 0 à 50 °C, à celui du cis-2-pentène-nitrile ou supérieur, c'est-à-dire supérieur de 1 à 50 °C, à celui du cis-3-pentène-nitrile, sont utilisés.

6. Procédé d'isomérisation de cis-2-pentène-nitrile en 3-pentène-nitriles selon l'une quelconque des revendications 1 à 5, **caractérisé en ce que** l'isomérisation du cis-2-pentène-nitrile en 3-pentène-nitriles est réalisée dans une colonne de réaction.

7. Procédé d'isomérisation continue de cis-2-pentène-nitrile en 3-pentène-nitriles selon la revendication 1, selon lequel
a) des 3-pentène-nitriles ou un mélange contenant des 3-pentène-nitriles est hydrocyané en adipodinitrile en présence de complexes de nickel(0)-ligand phosphore en tant que catalyseurs,
b) l'adipodinitrile, le 2-méthylglutarnitrile et le complexe de nickel(0)-ligand phosphore sont séparés de la sortie de l'hydrocyanation,
c) dans la phase organique contenant principalement des mononitriles en C5 insaturés, tels que des cis- et trans-3-pentène-nitriles, du 4-pentène-nitrile, du trans-2-pentène-nitrile, du cis-2-méthyl-2-butènenitrile, du trans-2-méthyl-2-butène-nitrile, du 2-méthyl-3-butène-nitrile, ainsi obtenue, le cis-2-pentène-nitrile est isomérisé en 3-pentène-nitriles à l'aide d'une base azotée de formule [R¹R²R³N], dans laquelle les radicaux R¹, R², R³ sont indépendamment les uns des autres un alkyle en C₁ à C₂₀, en tant que catalyseur, dont le point d'ébullition à pression normale est inférieur ou égal à celui du cis-2-pentène-nitrile, **caractérisé en ce que**
d) la phase organique contenant principalement des mononitriles en C5 insaturés obtenue en c) est introduite dans une colonne de distillation K1, du cis-2-pentène-nitrile, du cis- et trans-2-méthyl-2-butènenitrile sont séparés par la tête de la colonne de distillation K1, du 3-pentène-nitrile et du trans-2-pentène-nitrile sont séparés par le fond et recyclés dans l'étape de réaction de l'hydrocyanation des 3-pentène-nitriles,
e) le produit de tête de la colonne K1 est introduit dans une colonne de distillation K2, du trans-2-méthyl-2-butène-nitrile est séparé en tant que produit de fond de la colonne K2 et évacué,
f) le produit de tête de la colonne K2 et une base azotée ayant un point d'ébullition à pression normale inférieur ou égal à celui du cis-2-pentène-nitrile sont introduits dans un réacteur R1, le produit de réaction du réacteur R1 et l'amine tertiaire de point d'ébullition inférieur ou égal à celui du cis-2-pentène-nitrile sont introduits dans une colonne de distillation K3,
g) le produit de tête de la colonne de distillation K3, qui est principalement constitué de l'amine tertiaire, est recyclé dans le réacteur R1 et un courant partiel du produit de tête, qui contient du cis-2-méthyl-2-butène-nitrile, l'amine tertiaire et du cis-2-pentène-nitrile, est évacué, un mélange de cis-2-pentène-nitrile, de trans-3-pentène-nitrile, de trans-2-pentène-nitrile et de base tertiaire est recyclé à partir d'une sortie latérale dans la colonne de distillation K1 et des composants de point d'ébullition élevé sont évacués en tant que produit de fond.

8. Procédé d'isomérisation continue de cis-2-pentène-nitrile en 3-pentène-nitriles selon la revendication 7, **caractérisé en ce que** l'isomérisation de cis-2-pentène-nitrile en 3-pentène-nitriles est réalisée dans une colonne de réaction.

9. Utilisation d'amines tertiaires ou de leurs mélanges en tant que catalyseurs pour l'isomérisation de cis-2-pentène-nitrile en 3-pentène-nitrile.
